# EUROPEAN PATENT APPLICATION

(11) **EP 4 512 793 A1**
(43) Date of publication of application: **26.02.2025**
(21) Application number: 23791715.8
(22) Date of filing: 10.04.2023
(51) Int. Cl.: C07C 41/06, C07C 43/12, C07C 43/174, C07B 61/00

(54) **METHOD FOR PRODUCING FLUOROETHER**

(30) Priority: 20.04.2022 JP 2022069520
(71) Applicant: DAIKIN INDUSTRIES, LTD., Osaka-shi, Osaka 530-0001 (JP)
(72) Inventor: HOSHIYA, Naoyuki, Osaka-Shi, Osaka 530-0001 (JP); HAMADA, Tomohito, Osaka-Shi, Osaka 530-0001 (JP); YAMAUCHI, Akiyoshi, Osaka-Shi, Osaka 530-0001 (JP); KISHIKAWA, Yosuke, Osaka-Shi, Osaka 530-0001 (JP)
(74) Representative: Hoffmann Eitle
(86) International application number: PCT/JP2023/014486
(87) International publication number: WO 2023/204066

(57) **Abstract**

Disclosed is a method for producing a compound represented by formula (3), including step A of reacting a compound represented by formula (1) with a compound represented by formula (2) in the presence of at least one member selected from the group consisting of a compound represented by formula (4) and a compound represented by formula (5) 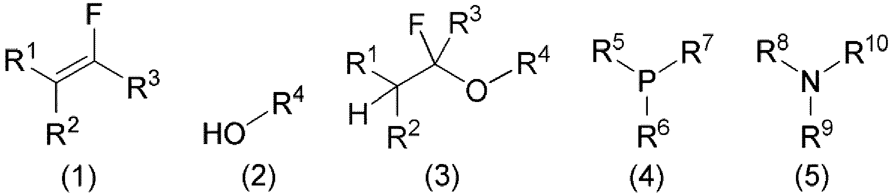 wherein R¹ to R¹⁰ are as defined for the symbols in the specification.

## Description

### Technical Field

The present disclosure relates to a method for producing a fluoroether.

### Background Art

Fluoroethers that have a fluorinated α-carbon (α-fluoroethers) are useful as a refrigerant, solvent, pharmaceutical, agrochemical, functional material, etc. The production method of α-fluoroethers can be broadly divided into two methods: etherification of a fluorine-containing compound, and fluorination of a hydrocarbon ether. The former method has advantages over the latter method in that mild reaction conditions are applicable, no special equipment is necessary, and it is easy to control the fluorine introduction position. An example of the former method is reacting a fluoroolefin with an alcohol. Known techniques of this method include methods using a strong base such as method 1, which uses KOH (e.g., PTL 1); and methods not using a strong base, such as method 2, which uses a palladium catalyst (e.g., PTL 2), and a method that uses an ionic liquid (e.g., PTL 3).

### Citation List

### Patent Literature

PTL 1: US Patent No. 3557294B
PTL 2: Japanese Patent No. 4009724B
PTL 3: JP2006-256967A

### Summary of Invention

### Technical Problem

Method 1 has room for improvement in terms of safety and environmental impact. Method 2 requires the use of the rare metal palladium, which is expensive and difficult to sustainably use. Method 3 requires the use of expensive ionic liquid, which is difficult to handle on an industrial scale.

A main object of the present disclosure is to provide a method for producing an α-fluoroether by using a predetermined phosphine and/or amine.

### Solution to Problem

The present disclosure includes the following aspects.

### Item 1

A method for producing a compound represented by the following formula (3): 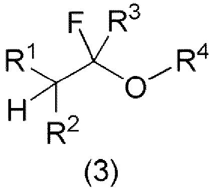 wherein
R¹ and R² each independently represent a hydrogen atom, a halogen atom, an alkyl group substituted with at least one fluorine atom, or an alkoxy group substituted with at least one fluorine atom,
R³ represents a fluorine atom, an alkyl group substituted with at least one fluorine atom, or an alkoxy group substituted with at least one fluorine atom,
R² and R³ may be bound together to form a ring, and
R⁴ represents an alkyl group optionally substituted with at least one substituent or an aryl group optionally substituted with at least one substituent,
the method comprising step A of reacting a compound represented by the following formula (1): 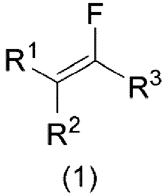 wherein R¹, R², and R³ are as defined above,
with a compound represented by the following formula (2) : 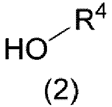 wherein R⁴ is as defined above,
in the presence of at least one member selected from the group consisting of
a compound represented by the following formula (4): 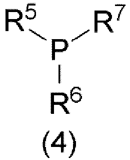 wherein R⁵, R⁶, and R⁷ each independently represent an organic group, and any two of R⁵, R⁶, and R⁷ may be bound together to form a ring, and
a compound represented by the following formula (5): 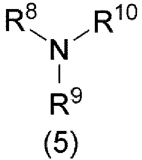 wherein R⁸, R⁹, and R¹⁰ each independently represent an organic group, and any two or all of R⁸, R⁹, and R¹⁰ may be bound together to form a ring.

### Item 2

The production method according to Item 1,
wherein
R⁵, R⁶, and R⁷ each independently represent an alkyl group optionally substituted with at least one substituent, an aryl group optionally substituted with at least one substituent, an alkoxy group optionally substituted with at least one substituent, or an aryloxy group optionally substituted with at least one substituent,
any two of R⁵, R⁶, and R⁷ may be bound together to form a ring,
R⁸, R⁹, and R¹⁰ each independently represent an alkyl group optionally substituted with at least one substituent or an aryl group optionally substituted with at least one substituent, and
any two or all of R⁸, R⁹, and R¹⁰ may be bound together to form a ring.

### Item 3

The production method according to Item 1 or 2, wherein the amount of the at least one member selected from the group consisting of the compound represented by formula (4) and the compound represented by formula (5) is 0.1 mol or less, per mole of the compound represented by formula (1).

### Item 4

The production method according to any one of Items 1 to 3, wherein R¹ and R² each independently represent a fluorine atom, an alkyl group substituted with at least one fluorine atom, or an alkoxy group substituted with at least one fluorine atom.

### Item 5

The production method according to any one of Items 1 to 4, wherein one of R¹ and R² is an alkyl group substituted with at least one fluorine atom or an alkoxy group substituted with at least one fluorine atom, and the other is a fluorine atom.

### Item 6

The production method according to any one of Items 1 to 5, wherein R³ is a fluorine atom.

### Item 7

The production method according to any one of Items 1 to 6, wherein step A is performed in the absence of a palladium catalyst or in the presence of a palladium catalyst in an amount of more than 0 mol and 0.01 mol or less, per mole of the compound represented by formula (1).

### Item 8

The production method according to any one of Items 1 to 7, wherein step A is performed in the presence of at least one solvent selected from the group consisting of ether solvents, sulfoxide solvents, nitrile solvents, and amide solvents.

### Item 9

A composition comprising
a compound represented by the following formula (3): 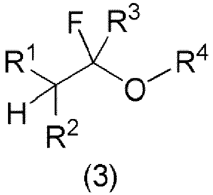 wherein
R¹ and R² each independently represent a hydrogen atom, a halogen atom, an alkyl group substituted with at least one fluorine atom, or an alkoxy group substituted with at least one fluorine atom,
R³ represents a fluorine atom, an alkyl group substituted with at least one fluorine atom, or an alkoxy group substituted with at least one fluorine atom,
R² and R³ may be bound together to form a ring, and
R⁴ represents an alkyl group optionally substituted with at least one substituent or an aryl group optionally substituted with at least one substituent; and
   water.

### Advantageous Effects of Invention

The present disclosure provides a method for producing an α-fluoroether, for example, by using a predetermined phosphine and/or amine.

### Description of Embodiments

The above overview of the present disclosure is not intended to describe each of the disclosed embodiments or all of the implementations of the present disclosure. The following description of the disclosure more specifically exemplifies the embodiments of examples.

In several parts of the present disclosure, guidance is provided through examples, and these examples can be used in various combinations.

In each case, the group of examples can function as a non-exclusive and representative group.

All publications, patents, and patent applications cited herein are incorporated herein by reference in their entirety.

### 1. Terms

Unless otherwise specified, the symbols and abbreviations in the present specification can be understood in the context of the present specification in the meanings commonly used in the technical field to which the present disclosure belongs.

In the present specification, the terms "comprise" and "contain" are used with the intention of including the terms "consisting essentially of" and "consisting of."

Unless otherwise specified, the steps, treatments, or operations described in the present specification can be performed at room temperature. In the present specification, room temperature can refer to a temperature within the range of 10 to 40°C.

In the present specification, the phrase "Cₙ₋ₘ" (wherein n and m are each a positive number, n < m) indicates that the number of carbon atoms is n or more and m or less.

In the present specification, examples of halogen atoms include a fluorine atom, a chlorine atom, a bromine atom, and an iodine atom.

In the present specification, the "organic group" refers to a group containing at least one carbon atom.

Examples of organic groups may include
a hydrocarbon group optionally substituted with at least one substituent,
a non-aromatic heterocyclic group optionally substituted with at least one substituent,
a heteroaryl group optionally substituted with at least one substituent,
a cyano group,
an aldehyde group,
a carboxyl group,

   R^{r}O-,

   R^{r}CO-,

   R^{r}COO-,

   R^{r}SO₂-,

   R^{r}OCO-,

   and

   R^{r}OSO₂-
wherein, R^{r} is independently
a hydrocarbon group optionally substituted with at least one substituent,
a non-aromatic heterocyclic group optionally substituted with at least one substituent, or
a heteroaryl group optionally substituted with at least one substituent).

In the present specification, the hydrocarbon group may be a saturated hydrocarbon group or an unsaturated hydrocarbon group. Examples of hydrocarbon groups include an alkyl group, an alkenyl group, an alkynyl group, a cycloalkyl group, a cycloalkenyl group, a cycloalkadienyl group, an aryl group, and a combination of two or more of these (e.g., an aralkyl group).

In the present specification, examples of alkyl groups include linear or branched C₁₋₁₂ alkyl groups, and specific examples thereof include a methyl group, an ethyl group, a propyl group (e.g., an n-propyl group and an isopropyl group), a butyl group (e.g., an n-butyl group, an isobutyl group, a sec-butyl group, and a tert-butyl group), a pentyl group, a hexyl group, a heptyl group, an octyl group, a nonyl group, and a decyl group.

In the present specification, examples of alkenyl groups include linear or branched C₂₋₂₀ alkenyl groups, and specific examples thereof include a vinyl group, a 1-propen-1-yl group, a 2-propen-1-yl group, an isopropenyl group, a 2-buten-1-yl group, a 4-penten-1-yl group, and a 5-hexen-1-yl group.

In the present specification, examples of alkynyl groups include linear or branched C₂₋₂₀ alkynyl groups, and specific examples thereof include an ethynyl group, a 1-propyn-1-yl group, a 2-propyn-1-yl group, a 4-pentyn-1-yl group, and a 5-hexyn-1-yl group.

In the present specification, examples of cycloalkyl groups include C₃₋₁₀ cycloalkyl groups, and specific examples thereof include a cyclopropyl group, a cyclobutyl group, a cyclopentyl group, a cyclohexyl group, and a cycloheptyl group.

In the present specification, examples of cycloalkenyl groups include C₃₋₁₀ cycloalkenyl groups, and specific examples thereof include a cyclopropenyl group, a cyclobutenyl group, a cyclopentenyl group, a cyclohexenyl group, and a cycloheptenyl group.

In the present specification, examples of cycloalkadienyl groups include C₄₋₁₀ cycloalkadienyl groups, and specific examples thereof include a cyclobutadienyl group, a cyclopentadienyl group, a cyclohexadienyl group, and a cycloheptadienyl group.

In the present specification, the aryl group may be a monocyclic or polycyclic (e.g., bicyclic, tricyclic, or quadracyclic) aryl group. Examples of aryl groups include C₆₋₁₄ aryl groups, and specific examples thereof include a phenyl group, a 1-naphthyl group, a 2-naphthyl group, a 2-biphenyl group, a 3-biphenyl group, a 4-biphenyl group, and a 2-anthryl group.

In the present specification, the heteroaryl group includes monocyclic aromatic heterocyclic groups (e.g., 5- or 6-membered monocyclic aromatic heterocyclic groups) and aromatic condensed heterocyclic groups (e.g., 5- to 18-membered aromatic condensed heterocyclic groups).

In the present specification, examples of 5- or 6-membered monocyclic aromatic heterocyclic groups include pyrrolyl (e.g., 1-pyrrolyl, 2-pyrrolyl, and 3-pyrrolyl), furyl (e.g., 2-furyl and 3-furyl), thienyl (e.g., 2-thienyl and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-4-yl and 1,2,4-triazol-3-yl), oxadiazolyl (e.g., 1,2,4-oxadiazol-3-yl and 1,2,4-oxadiazol-5-yl), thiadiazolyl (e.g., 1,2,4-thiadiazol-3-yl and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), and pyrazinyl.

In the present specification, examples of 5- to 18-membered aromatic condensed heterocyclic groups include isoindolyl (e.g., 1-isoindolyl, 2-isoindolyl, 3-isoindolyl, 4-isoindolyl, 5-isoindolyl, 6-isoindolyl, and 7-isoindolyl), indolyl (e.g., 1-indolyl, 2-indolyl, 3-indolyl, 4-indolyl, 5-indolyl, 6-indolyl, and 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, and 7-benzo[b]furanyl), benzo[c]furanyl (e.g., 1-benzo[c]furanyl, 4-benzo[c]furanyl, and 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, and 7-benzo[b]thienyl), benzo[c]thienyl (e.g., 1-benzo[c]thienyl, 4-benzo[c]thienyl, and 5-benzo[c]thienyl), indazolyl (e.g., 1-indazolyl, 2-indazolyl, 3-indazolyl, 4-indazolyl, 5-indazolyl, 6-indazolyl, and 7-indazolyl), benzimidazolyl (e.g., 1-benzimidazolyl, 2-benzimidazolyl, 4-benzimidazolyl, and 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, and 1,2-benzisoxazol-7-yl), benzoxazolyl (e.g., 2-benzoxazolyl, 4-benzoxazolyl, 5-benzoxazolyl, 6-benzoxazolyl, and 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, and 1,2-benzisothiazol-7-yl), benzothiazolyl (e.g., 2-benzothiazolyl, 4-benzothiazolyl, 5-benzothiazolyl, 6-benzothiazolyl, and 7-benzothiazolyl), isoquinolyl (e.g., 1-isoquinolyl, 3-isoquinolyl, 4-isoquinolyl, and 5-isoquinolyl), quinolyl (e.g., 2-quinolyl, 3-quinolyl, 4-quinolyl, 5-quinolyl, and 8-quinolyl), cinnolinyl (e.g., 3-cinnolinyl, 4-cinnolinyl, 5-cinnolinyl, 6-cinnolinyl, 7-cinnolinyl, and 8-cinnolinyl), phthalazinyl (e.g., 1-phthalazinyl, 4-phthalazinyl, 5-phthalazinyl, 6-phthalazinyl, 7-phthalazinyl, and 8-phthalazinyl), quinazolinyl (e.g., 2-quinazolinyl, 4-quinazolinyl, 5-quinazolinyl, 6-quinazolinyl, 7-quinazolinyl, and 8-quinazolinyl), quinoxalinyl (e.g., 2-quinoxalinyl, 3-quinoxalinyl, 5-quinoxalinyl, 6-quinoxalinyl, 7-quinoxalinyl, and 8-quinoxalinyl), pyrazolo[1,5-a]pyridyl (e.g., pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, and pyrazolo[1,5-a]pyridin-7-yl), imidazo[1,2-a]pyridyl (e.g., imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl).

In the present specification, -CH₂- that can be present in the hydrocarbon group of the "hydrocarbon group optionally substituted with at least one substituent" may be replaced with -O-, -S-, or -NH-. Examples of substituents for the hydrocarbon group include a halogen atom, a hydroxy group, a mercapto group, an amino group, a nitro group, and a cyano group. If the hydrocarbon group has substituents, the number of substituents can be selected from one to the maximum number of substituents possible, such as one, two, three, four, or five. If the number of substituents is two or more, the substituents may be identical or different from each other.

In the present specification, examples of substituents for the alkyl group optionally substituted with at least one substituent include a halogen atom, a hydroxy group, R^{A}O- (wherein R^{A} represents an alkyl group or an aryl group), (R^{B})₂N- (wherein each R^{B} independently represents a hydrogen atom, an alkyl group, or an aryl group), (R^{C})₂P- (wherein each R^{c} independently represents a hydrogen atom, an alkyl group, or an aryl group), and a combination of two or more of these (e.g., a haloalkoxy group). If the alkyl group has substituents, the number of substituents can be selected from one to the maximum number of substituents possible, such as one, two, three, four, or five. If the number of substituents is two or more, the substituents may be identical or different from each other.

Examples of alkyl groups optionally substituted with at least one substituent include alkyl groups optionally substituted with at least one fluorine atom. The alkyl group substituted with at least one fluorine atom may be referred to as a "fluoroalkyl group." The fluoroalkyl group may be a perfluoroalkyl group or non-perfluoroalkyl group. Examples of fluoroalkyl groups include linear or branched C₁₋₁₂ fluoroalkyl groups, and specific examples thereof include linear or branched C₁₋₁₂ fluoroalkyl groups, such as a trifluoromethyl group, a perfluoroethyl group, a perfluoropropyl group (e.g., a perfluoro-n-propyl group, and a perfluoroisopropyl group), a perfluorobutyl group, a perfluoropentyl group, and a perfluorohexyl group. The alkyl group includes those having at least one substituent other than a fluorine atom (e.g., a fluoroalkoxy group), such as CF₃-O-CF₂-, CF₃-O-CF(CF₃)-, CF₃-O-CH₂-CH₂-, CF₃-O-CH(CF₃)-CH₂-, CF₃-O-CF₂-CF₂-, CF₃-CF₂-O-CF₂-, CF₃-CF₂-O-CF₂-CF₂-, CF₃-O-CF₂-O-CF₂-, CF₃-CF₂-CF₂-O-CH₂-CF₂-, CF₃-CF₂-CF₂-O-CF₂-CF₂-, CF₃-CF₂-CF₂-O-CF(CF₃)-CF₂-, CF₃-CF₂-CF₂-O-CF(CF₃)-CF₂-O-CF(CF₃)-CF₂-, and CF₃-CF₂-CF₂-O-[CF(CF₃)-CF₂-O-]₂-CF(CF₃)-CF₂-.

In the present specification, examples of substituents for the aryl group optionally substituted with at least one substituent include a halogen atom, a hydroxy group, an alkyl group, R^{A}O- (wherein R^{A} represents an alkyl group or an aryl group), (R^{B})₂N- (wherein each R^{B} independently represents a hydrogen atom, an alkyl group, or an aryl group), (R^{C})₂P- (wherein each R^{C} independently represents a hydrogen atom, an alkyl group, or an aryl group), and a combination of two or more of these (e.g., a haloalkyl group). If the aryl group has substituents, the number of substituents can be selected from one to the maximum number of substituents possible, such as one, two, three, four, or five. If the number of substituents is two or more, the substituents may be identical or different from each other.

In the present specification, examples of alkoxy groups include linear or branched C₁₋₁₂ alkoxy groups, and specific examples thereof include a methoxy group, an ethoxy group, a propoxy group (e.g., an n-propoxy group and an isopropoxy group), a butoxy group, a pentyloxy group, and a hexyloxy group.

In the present specification, examples of substituents for the alkoxy group optionally substituted with at least one substituent include a halogen atom, a hydroxy group, an alkyl group, an alkoxy group, and a combination of two or more of these (e.g., a haloalkyl group and a haloalkoxy group). If the alkoxy group has substituents, the number of substituents can be selected from one to the maximum number of substituents possible, such as one, two, three, four, or five. If the number of substituents is two or more, the substituents may be identical or different from each other.

Examples of alkoxy groups optionally substituted with at least one substituent include alkoxy groups optionally substituted with at least one fluorine atom. The alkoxy group substituted with at least one fluorine atom may be referred to as a "fluoroalkoxy group." The fluoroalkoxy group may be a perfluoroalkoxy group or a non-perfluoroalkoxy group. Examples of fluoroalkoxy groups include linear or branched C₁₋₁₂ fluoroalkoxy groups, and specific examples thereof include linear or branched C₁₋₁₂ fluoroalkoxy groups, such as a perfluoromethoxy group, a perfluoroethoxy group, a perfluoropropoxy group (e.g., a perfluoro-n-propoxy group and a perfluoroisopropoxy group), a perfluorobutoxy group, a perfluoropentyloxy group, and a perfluorohexyloxy group. The alkoxy group includes those having at least one substituent other than a fluorine atom (e.g., a fluoroalkoxy group), such as CF₃-O-CF₂-O-, CF₃-O-CF₂-CF₂-CF₂-O-, CF₃-O-CH₂-CF₂-CF₂-O-, CF₃-CF₂-O-CF₂-CF₂-O-, CF₃-CF₂-CF₂-O-CH₂-CF₂-CF₂-O-, CF₃-CF₂-CF₂-O-CF₂-CF₂-CF₂-O-, CF₃-CF₂-CF₂-O-CF(CF₃)-CF₂-O-, CF₃-CF₂-CF₂-O-[CF(CF₃)-CF₂-O-]₂-, and CF₃-CF₂-CF₂-O-[CF(CF₃)-CF₂-O-]₃-.

In the present specification, examples of aryloxy groups include C₆₋₁₂ aryloxy groups, and specific examples thereof include a phenoxy group and a naphthoxy group.

In the present specification, examples of substituents for the aryloxy group optionally substituted with at least one substituent include a halogen atom, a hydroxy group, an alkyl group, an alkoxy group, and a combination of two or more of these (e.g., a haloalkyl group and a haloalkoxy group). If the aryloxy group has substituents, the number of substituents can be selected from one to the maximum number of substituents possible, such as one, two, three, four, or five. If the number of substituents is two or more, the substituents may be identical or different from each other.

### 2. Method for Producing α-Fluoroether

In an embodiment of the present disclosure, a method for producing a compound represented by the following formula (3) : 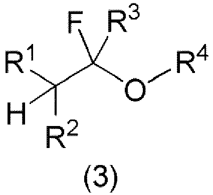 wherein
R¹ and R² each independently represent a hydrogen atom, a halogen atom, an alkyl group substituted with at least one fluorine atom, or an alkoxy group substituted with at least one fluorine atom,
R³ represents a fluorine atom, an alkyl group substituted with at least one fluorine atom, or an alkoxy group substituted with at least one fluorine atom,
R² and R³ may be bound together to form a ring, and
R⁴ represents an alkyl group optionally substituted with at least one substituent or an aryl group optionally substituted with at least one substituent,
comprises step A of reacting a compound represented by the following formula (1): 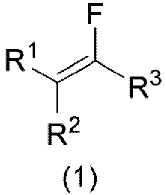 wherein R¹, R², and R³ are as defined above,
with a compound represented by the following formula (2) : 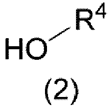 wherein R⁴ is as defined above,
in the presence of at least one member selected from the group consisting of
a compound represented by the following formula (4): 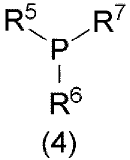 wherein R⁵, R⁶, and R⁷ each independently represent an organic group, and any two of R⁵, R⁶, and R⁷ may be bound together to form a ring, and
a compound represented by the following formula (5): 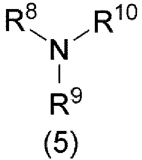 wherein R⁸, R⁹, and R¹⁰ each independently represent an organic group, and any two of R⁸, R⁹, and R¹⁰ may be bound together to form a ring.

### Compound Represented by Formula (1)

R¹ and R² each independently represent preferably a halogen atom, an alkyl group substituted with at least one fluorine atom, or an alkoxy group substituted with at least one fluorine atom, more preferably a fluorine atom, an alkyl group substituted with at least one fluorine atom, or an alkoxy group substituted with at least one fluorine atom, and still more preferably a fluorine atom or an alkyl group substituted with at least one fluorine atom.

R¹ and R² may both be a halogen atom (e.g., one is a chlorine atom or a bromine atom, and the other is a fluorine atom), or R¹ and R² may both be an alkyl group substituted with at least one fluorine atom or an alkoxy group substituted with at least one fluorine atom; however, it is preferred that one of R¹ and R² be an alkyl group substituted with at least one fluorine atom or an alkoxy group substituted with at least one fluorine atom, with the other being a fluorine atom. In particular, it is preferred that one of R¹ and R² be an alkyl group substituted with at least one fluorine atom, with the other being a fluorine atom.

If R¹ or R² is an alkyl group substituted with at least one fluorine atom, the alkyl group substituted with at least one fluorine atom is preferably a C₁₋₁₀ fluoroalkyl group, such as a trifluoromethyl group, a perfluoroethyl group, a perfluoropropyl group, or a perfluorobutyl group, or a (C₁₋₄ fluoroalkoxy) C₁₋₄ fluoroalkyl group, such as CF₃-O-CF₂- or CF₃-O-(CF₂)₂-. If R¹ or R² is an alkoxy group substituted with at least one fluorine atom, the alkoxy group substituted with at least one fluorine atom is preferably a C₁₋₁₀ fluoroalkoxy group, such as a trifluoromethoxy group, a perfluoroethoxy group, a perfluoropropoxy group, or a perfluorobutoxy group, or a (C₁₋₄ fluoroalkoxy) C₁₋₄ fluoroalkoxy group, such as CF₃-O-CF₂-O- or CF₃-O-(CF₂)₂-O-.

R³ is preferably a fluorine atom or an alkyl group substituted with at least one fluorine atom, more preferably a fluorine atom, a C₁₋₁₀ fluoroalkyl group, or a (C₁₋₄ fluoroalkoxy) C₁₋₄ fluoroalkyl group, and still more preferably a fluorine atom.

If R² and R³ are bound together to form a ring, examples of rings include cyclobutene, cyclopentene, cyclohexene, cycloheptene, and cyclooctene. The ring may have at least one substituent (e.g., a halogen atom, such as a fluorine atom).

### Compound Represented by Formula (2)

A preferred example of R⁴ is an alkyl group optionally substituted with at least one substituent, and R⁴ is more preferably a group represented by the following formula (A): 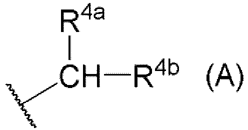 wherein R^{4a} and R^{4b} each independently represent a hydrogen atom, an alkyl group optionally substituted with at least one substituent, or an aryl group optionally substituted with at least one substituent, and the wavy line represents a binding point at which a hydroxy group binds.

The group represented by formula (A) may be any of the following (A-1) to (A-5):
(A-1) R^{4a} and R^{4b} are both a hydrogen atom,
(A-2) R^{4a} is a hydrogen atom, and R^{4b} is an alkyl group optionally substituted with at least one substituent or an aryl group optionally substituted with at least one substituent,
(A-3) R^{4a} and R^{4b} are both an alkyl group optionally substituted with at least one substituent,
(A-4) R^{4a} is an alkyl group optionally substituted with at least one substituent, and R^{4b} is an aryl group optionally substituted with at least one substituent, and
(A-5) R^{4a} and R^{4b} are both an aryl group optionally substituted with at least one substituent.

If R^{4a} and/or R^{4b} is an alkyl group, the alkyl group may be, for example, a C₁₋₁₀ alkyl group, and may be substituted with at least one (e.g., one, two, three, four, or five) substituent selected from the group consisting of a halogen atom (e.g., a fluorine atom), R^{A}O-, (R^{B})₂N-, (R^{C})₂P-, an aryl group (e.g., a C₆₋₁₂ aryl group, such as a phenyl group), and a combination of two or more of these.

If R^{4a} and/or R^{4b} is an aryl group, the aryl group may be, for example, a C₆₋₁₂ alkyl group, such as a phenyl group and may be substituted with at least one (e.g., one, two, three, four, or five) substituent selected from the group consisting of a halogen atom, an alkyl group (e.g., a C₁₋₁₀ alkyl group), R^{A}O-, (R^{B})₂N-, (R^{C})₂P-, and a combination of two or more of these.

Another preferred example of R⁴ is an aryl group optionally substituted with at least one substituent. R⁴ is more preferably a C₆₋₁₂ aryl group optionally substituted with at least one substituent, and still more preferably a phenyl group optionally substituted with at least one substituent. The aryl group may be substituted with, for example, at least one (e.g., one, two, three, four, or five) substituent selected from the group consisting of a halogen atom, an alkyl group (e.g., a C₁₋₁₀ alkyl group), R^{A}O-, (R^{B})₂N-, (R^{C})₂P-, and a combination of two or more of these.

The amount of the compound represented by formula (2) for use is, although not limited to, for example, 0.1 mol or more, 0.2 mol or more, 0.3 mol or more, 0.4 mol or more, or 0.5 mol or more, and 5 mol or less, 4 mol or less, 3 mol or less, 2 mol or less, 1.5 mol or less, or 1 mol or less, or may be a substantially equimolar amount, such as 0.5 to 1.5 mol, per mole of the compound represented by formula (1).

### Compound Represented by Formula (4)

R⁵, R⁶, and R⁷ each independently represent preferably a hydrocarbon group optionally substituted with at least one substituent, a heteroaryl group optionally substituted with at least one substituent (e.g., a furyl group and a thienyl group), or R^{X}-O- (wherein R^{X} represents a hydrocarbon group optionally substituted with at least one substituent); more preferably a hydrocarbon group optionally substituted with at least one substituent or R^{X}-O-; still more preferably an alkyl group optionally substituted with at least one substituent, a cycloalkyl group optionally substituted with at least one substituent, an aryl group optionally substituted with at least one substituent, an alkoxy group optionally substituted with at least one substituent, or an aryloxy group optionally substituted with at least one substituent; yet more preferably an alkyl group optionally substituted with at least one substituent, an aryl group optionally substituted with at least one substituent, an alkoxy group optionally substituted with at least one substituent, or an aryloxy group optionally substituted with at least one substituent; and particularly preferably a C₁₋₆ alkyl group optionally substituted with at least one substituent, a C₆₋₁₂ aryl group optionally substituted with at least one substituent (e.g., a phenyl group, a tolyl group, and a biphenyl group), a C₁₋₆ alkoxy group optionally substituted with at least one substituent, or a C₆₋₁₂ aryloxy group optionally substituted with at least one substituent (e.g., a phenoxy group). Preferred examples of substituents include a halogen atom, R^{A}O-, and (R^{C})₂P-, and more preferred examples include a C₁₋₄ alkoxy group, such as a methoxy group, and a C₆₋₁₂ diarylphosphino group, such as a diphenylphosphino group.

If any two of R⁵, R⁶, and R⁷ are bound together to form a ring, the ring is, for example, a phosphetane, phosphorane, phosphorinan, or phosphepane.

Although the compound represented by formula (4) may be a phosphine ligand capable of coordinating to a transition metal (e.g., Pd), the production method of the present disclosure can be suitably performed in the absence of, or substantially in the absence of, a transition metal.

Examples of the compound represented by formula (4) include tri-n-butylphosphine, tri-t-butylphosphine, di-t-butylphenylphosphine, methyl diphenylphosphine, ethyl diphenylphosphine, isopropyl diphenylphosphine, 2-(di-t-butylphosphino)biphenyl, bis(diphenylphosphino)methane, 1,2-bis(diphenylphosphino)ethane, 1,3-bis(diphenylphosphino)propane, 1,4-bis(diphenylphosphino)butane, 1,5-bis(diphenylphosphino)pentane, 1,6-bis(diphenylphosphino)hexane, tricyclohexylphosphine, dicyclohexylphenylphosphine, dicyclohexyl(2',4',6'-triisopropyl-[1,1'-biphenyl]-2-yl)phosphine (XPhos), triphenylphosphine, 4-(dimethylamino)phenyl diphenylphosphine, tritolylphosphine, tris(4-fluorophenyl)phosphine, tris[3,5-bis(trifluoromethyl)phenyl]phosphine, tris(4-methoxyphenyl)phosphine, tris(2,6-dimethoxyphenyl)phosphine, 2-(diphenylphosphino)biphenyl, 2-diphenylphosphino-2'-methoxy-1,1'-binaphthyl, 2,2'-bis(diphenylphosphino)-1,1'-binaphthyl, bis [2-(diphenylphosphino)phenyl]ether, tri(2-furyl)phosphine, tri(2-thienyl)phosphine, triphenyl phosphite, triethyl phosphite, and triisopropyl phosphite.

### Compound Represented by Formula (5)

R⁸, R⁹, and R¹⁰ each independently represent preferably a hydrocarbon group optionally substituted with at least one substituent or a heteroaryl group optionally substituted with at least one substituent (e.g., a pyridyl group); more preferably a hydrocarbon group optionally substituted with at least one substituent; still more preferably an alkyl group optionally substituted with at least one substituent, a cycloalkyl group optionally substituted with at least one substituent, or an aryl group optionally substituted with at least one substituent; yet more preferably an alkyl group optionally substituted with at least one substituent or an aryl group optionally substituted with at least one substituent; and particularly preferably a C₁₋₆ alkyl group optionally substituted with at least one substituent or a C₆₋₁₂ aryl group optionally substituted with at least one substituent (e.g., a phenyl group). Preferred examples of substituents include R^{A}O-, (R^{B})₂N-, and (R^{C})₂P-, and more preferred examples of substituents include a C₁₋₄ alkoxy group, such as a methoxy group, a C₁₋₄ dialkylamino group, such as a dimethylamino group, and a C₆₋₁₂ diarylphosphino group, such as a diphenylphosphino group.

If any two or all of R⁸, R⁹, and R¹⁰ are bound together to form a ring, the ring is, for example, pyridine, imidazole, morpholine, diazabicyclononene, or diazabicycloundecene.

Although the compound represented by formula (5) may be an amine ligand capable of coordinating to a transition metal (e.g., Pd), the production method of the present disclosure can be suitably performed in the absence of, or substantially in the absence of, a transition metal.

Examples of the compound represented by formula (5) include triethylamine, diisopropylethylamine, tri-n-propylamine, triisopropylamine, pyridine, 4-dimethylaminopyridine, 1-methylimidazole, N,N,N,N-tetramethyl-1,3-diaminopropane, N,N,N,N-tetraethylethylenediamine, N,N,N,N-tetramethyl-1,4-diaminobutane, 1-(diphenylphosphino)-N,N-dimethyl-2-propaneamine, diazabicyclononene, and diazabicycloundecene.

The amount of the at least one member selected from the group consisting of the compound represented by formula (4) and the compound represented by formula (5) for use is, although not limited to, preferably a catalytic amount. Specifically, the amount is preferably 0.5 mol or less, more preferably 0.4 mol or less, still more preferably 0.3 mol or less, yet more preferably 0.2 mol or less, and particularly preferably 0.1 mol or less, per mole of the compound represented by formula (1). The amount may be, for example, 0.0001 mol or more, 0.0005 mol or more, or 0.001 mol or more, or within the range of 0.0001 to 0.5 mol, 0.0005 to 0.2 mol, or 0.001 to 0.1 mol, per mole of the compound represented by formula (1).

### Solvent

Step A is preferably performed in the presence of a solvent. Examples of solvents include hydrocarbon solvents, halogen solvents, ether solvents, nitrile solvents, amide solvents, ketone solvents, and sulfoxide solvents.

Examples of hydrocarbon solvents include aliphatic hydrocarbon solvents, such as hexane, heptane, octane, nonane, and decane, and aromatic hydrocarbon solvents, such as benzene, toluene, and xylene.

Examples of halogen solvents include dichloromethane, dichloroethane, chloroform, and chlorobenzene.

Examples of ether solvents include diethyl ether, diisopropyl ether, ethylene glycol dimethyl ether (DME), diethylene glycol dimethyl ether (diglyme), triethylene glycol dimethyl ether (triglyme), tetraethylene glycol dimethyl ether (tetraglyme), 1,4-dioxane, and tetrahydrofuran.

Examples of nitrile solvents include acetonitrile, propionitrile, and benzonitrile.

Examples of amide solvents include formamide, N,N-dimethylformamide, and N-methylpyrrolidone.

Examples of ketone solvents include acetone, and methyl ethyl ketone.

Examples of sulfoxide solvents include dimethyl sulfoxide.

Of these solvents, at least one member selected from the group consisting of ether solvents, sulfoxide solvents, nitrile solvents, and amide solvents is preferable.

The amount of the solvent for use may be, although not limited to, for example, 0.1 mL or more, 0.15 mL or more, 0.2 mL or more, 0.25 mL or more, 0.5 mL or more, 1 mL or more, or 1.5 mL or more, and 5 mL or less, 3 mL or less, 2.5 mL or less, or 2 mL or less, and may be within the range of 0.2 mL to 3 mL, per millimole of the compound represented by formula (2).

### Transition Metal Catalyst

Step A is preferably performed in the absence of a transition metal catalyst or in the presence of a transition metal catalyst in an amount of more than 0 mol and 0.01 mol or less, per mole of the compound represented by formula (1). The transition metal catalyst may be, for example, palladium. In an embodiment, step A is performed in the absence of a palladium catalyst or in the presence of a palladium catalyst in an amount of more than 0 mol and 0.01 mol or less, per mole of the compound represented by formula (1).

### Inorganic Base

Step A is preferably performed in the absence of an inorganic base or in the presence of an inorganic base in an amount of more than 0 mol and 2 mol or less, per mole of the compound represented by formula (2). Examples of inorganic bases include alkali metal hydroxides, such as KOH and NaOH, and alkaline earth metal hydroxides, such as Ca(OH)₂. In an embodiment, step A is preferably performed in the absence of an alkali metal hydroxide or alkaline earth metal hydroxide or in the presence of an alkali metal hydroxide or alkaline earth metal hydroxide in an amount of more than 0 mol and 2 mol or less, per mole of the compound represented by formula (2).

### Ionic Liquid

Step A is preferably performed in the absence of an ionic liquid or in the presence of an ionic liquid in an amount of more than 0 mL and less than 0.1 mL, per mole of the compound represented by formula (1). Examples of ionic liquids include imidazolium salts, such as 1-ethyl-3-methylimidazolium hexafluorophosphate and 1-butyl-3-methylimidazolium chloride, and pyridinium salts. In an embodiment, step A is preferably performed in the absence of an imidazolium salt or a pyridinium salt or in the presence of an imidazolium salt or a pyridinium salt in an amount of more than 0 mL and less than 0.1 mL, per mole of the compound represented by formula (1).

### Reaction Temperature and Reaction Time

The reaction temperature and reaction time of step A are not limited as long as the reaction proceeds. The reaction temperature may be, for example, 0°C to 60°C. The reaction time may be within the range of, for example, 10 minutes to 72 hours, and preferably 15 minutes to 48 hours.

### 3. Composition

The composition according to an embodiment of the present disclosure contains the compound represented by formula (3) and water. The amount of the compound represented by formula (3) may be, although not limited to, for example, 60 parts by mass or more, 65 parts by mass or more, 70 parts by mass or more, or 73 parts by mass or more, and less than 100 parts by mass, 99.99 parts by mass or less, 99.9 parts by mass or less, 99 parts by mass or less, or 95 parts by mass or less, or may be within the range of 60 to 95 parts by mass, per 100 parts by mass of the composition. The amount can be measured, for example, according to ¹H-NMR, gas chromatography (GC), or gas chromatography-mass spectrometry (GC-MS).

The amount of water may be, although not limited to, for example, 0.3 parts by mass or less, 0.2 parts by mass or less, 0.1 parts by mass or less, 0.05 parts by mass or less, 0.02 parts by mass or less, 0.01 parts by mass or less, or 0.001 parts by mass or less, and more than 0 parts by mass, 0.0001 parts by mass or more, 0.0002 parts by mass or more, or 0.0003 parts by mass or more, or may be within the range of 0.0003 to 0.001 parts by mass, per 100 parts by mass of the composition or per 100 parts by mass of the compound represented by formula (3). The amount can be measured, for example, according to the Karl Fischer method.

The composition may further contain other optional components. Examples of other optional components include byproducts from the reaction in step A.

### Examples

The following describes in more detail an embodiment of the present disclosure with reference to Examples. However, the disclosure is not limited to these Examples.

In a nitrogen atmosphere, 2.0 mg of triphenylphosphine, 1.17 g of methanol, and 18 mL of acetonitrile were added to a 30 mL autoclave. The autoclave was depressurized at 0°C, and 5 g of 1,1,1,2,3,3-hexafluoropropene was added thereto, followed by stirring at room temperature for 19 hours. 200 mg of hexafluorobenzene (internal standard) was added to the autoclave, and analysis was performed according to 19F NMR, which found the formation of a fluoroether represented by formula (3-1) in a yield of 89%. After extraction with perfluorohexane, the solvent was distilled off, thereby giving a fluoroether represented by formula (3-1) in a yield of 90%. Measurement according to gas chromatography found that the compounds represented by formulas (6), (7), and (8) were present in a total amount of 27 mass%. The water value measured according to Karl Fischer was 6.6 ppm.

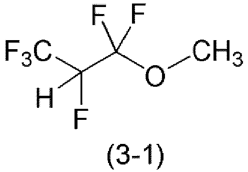

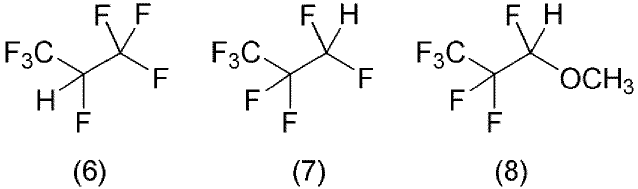

The same operation was performed by using the catalysts shown in Table 1 instead of triphenylphosphine, and stirring was performed at room temperature for 19 hours, thereby forming fluoroethers of formula (3-1) in the yields shown in Table 1.

**Table 1**

| Catalyst | Catalytic Amount (Equivalent amount relative to Methanol) | Yield of Compound (3-1) |
|---|---|---|
| nBu₃P | 0.0005 | 95% |
| Cy₃P | 0.0005 | 93% |
| tBu₃P | 0.0005 | 93% |
| PEtPh₂ | 0.0005 | 99% |
| (O-CH₃C₆H₄)₃P | 0.0003 | 61% |
| Ph₂P(CH₂)₄PPh₂ | 0.000075 | 97% |
| (EtO)₃P | 0.01 | 87% |
| 4-Dimethylaminopyridine | 0.0005 | 95% |

In Table 1, nBu denotes n-butyl, Cy denotes cyclohexyl, tBu denotes tert-butyl, Et denotes ethyl, and Ph denotes phenyl.

The same operation was performed by using 43.7 mg of triphenylphosphine, 2.24 g of 2-phenylethan-1-ol, 2.5 g of 1,1,1,2,3,3-hexafluoropropene, and 9 mL of acetonitrile, and stirring was performed at room temperature for 17 hours, thereby forming a fluoroether represented by formula (3-2) in a yield of 76%. After purification according to silica gel column chromatography, a fluoroether represented by formula (3-2) was obtained in a yield of 80%. The water value measured according to Karl Fischer was 3.9 ppm.

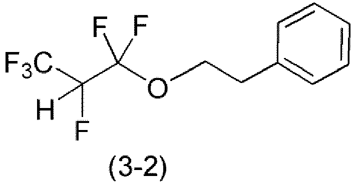

The same operation was performed by using 48.1 mg of triphenylphosphine, 2.13 g of heptan-4-ol, 3.03 g of 1,1,1,2,3,3-hexafluoropropene, and 9 mL of acetonitrile, and stirring was performed at 50°C for 22 hours, followed by removing the solvent, thereby forming a fluoroether represented by formula (3-3) in a yield of 38%. The water value measured according to Karl Fischer was 93 ppm.

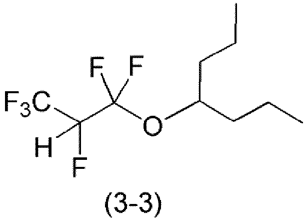

The same operation was performed by using 48.1 mg of triphenylphosphine, 2.76 g of 4-(tert-butyl)phenol, 3.03 g of 1,1,1,2,3,3-hexafluoropropene, and 9 mL of acetonitrile, and stirring was performed at 50°C for 22 hours, thereby forming a fluoroether represented by formula (3-4) in a yield of 97%.

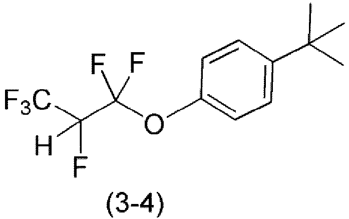

In a nitrogen atmosphere, 66.8 mg of tri-tert-butylphosphine, 1.17 g of methanol, and 18 mL of acetonitrile were added to a 30 mL autoclave. The autoclave was depressurized at 0°C, and 3.30 g of 1,1,2,2-tetrafluoroethylene was added thereto, followed by stirring at room temperature for 36 hours. 200 mg of hexafluorobenzene (internal standard) was added to the autoclave, and analysis was performed according to 19F NMR, which found the formation of a fluoroether represented by formula (3-5) in a yield of 79%.

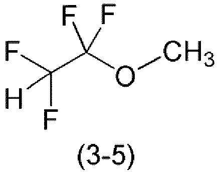

The same operation was performed by using 20.2 mg of 4-dimethylaminopyridine instead of tri-tert-butylphosphine, and stirring was performed at room temperature for 36 hours, thereby forming a fluoroether represented by formula (3-5) in a yield of 99%.

In a nitrogen atmosphere, 38.0 mg of dimethylphenylphosphine, 2.24 g of 2-phenylethan-1-ol, and 9 mL of acetonitrile were added to a 30 mL autoclave. The autoclave was depressurized at 0°C, and 1.83 g of 1,1,2,2-tetrafluoroethylene was added thereto, followed by stirring at 50°C for 24 hours. 161 mg of hexafluorobenzene (internal standard) was added to the autoclave, and analysis was performed according to 19F NMR, which found the formation of a fluoroether represented by formula (3-6) in a yield of 98%. After purification according to silica gel column chromatography, a fluoroether represented by formula (3-6) was obtained in a yield of 95%. The water value measured according to Karl Fischer was 0.13%.

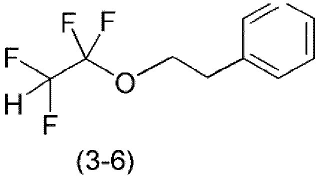

The same operation was performed by using 33.6 mg of 4-dimethylaminopyridine instead of dimethylphenylphosphine, and stirring was performed at 50°C for 24 hours, thereby forming a fluoroether represented by formula (3-6) in a yield of 96%.

## Claims

1. A method for producing a compound represented by the following formula (3): 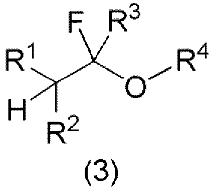 wherein
R¹ and R² each independently represent a hydrogen atom, a halogen atom, an alkyl group substituted with at least one fluorine atom, or an alkoxy group substituted with at least one fluorine atom,
R³ represents a fluorine atom, an alkyl group substituted with at least one fluorine atom, or an alkoxy group substituted with at least one fluorine atom,
R² and R³ may be bound together to form a ring, and
R⁴ represents an alkyl group optionally substituted with at least one substituent or an aryl group optionally substituted with at least one substituent,
the method comprising step A of reacting a compound represented by the following formula (1): 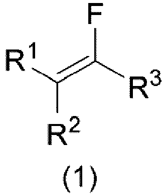 wherein R¹, R², and R³ are as defined above,
with a compound represented by the following formula (2) : 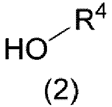 wherein R⁴ is as defined above,
in the presence of at least one member selected from the group consisting of
a compound represented by the following formula (4): 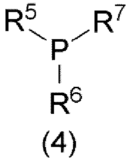 wherein R⁵, R⁶, and R⁷ each independently represent an organic group, and any two of R⁵, R⁶, and R⁷ may be bound together to form a ring, and
a compound represented by the following formula (5): 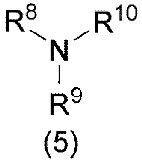 wherein R⁸, R⁹, and R¹⁰ each independently represent an organic group, and any two or all of R⁸, R⁹, and R¹⁰ may be bound together to form a ring.

2. The production method according to claim 1,
wherein
R⁵, R⁶, and R⁷ each independently represent an alkyl group optionally substituted with at least one substituent, an aryl group optionally substituted with at least one substituent, an alkoxy group optionally substituted with at least one substituent, or an aryloxy group optionally substituted with at least one substituent,
any two of R⁵, R⁶, and R⁷ may be bound together to form a ring,
R⁸, R⁹, and R¹⁰ each independently represent an alkyl group optionally substituted with at least one substituent or an aryl group optionally substituted with at least one substituent, and
any two or all of R⁸, R⁹, and R¹⁰ may be bound together to form a ring.

3. The production method according to claim 1 or 2, wherein the amount of the at least one member selected from the group consisting of the compound represented by formula (4) and the compound represented by formula (5) is 0.1 mol or less, per mole of the compound represented by formula (1).

4. The production method according to any one of claims 1 to 3, wherein R¹ and R² each independently represent a fluorine atom, an alkyl group substituted with at least one fluorine atom, or an alkoxy group substituted with at least one fluorine atom.

5. The production method according to any one of claims 1 to 4, wherein one of R¹ and R² is an alkyl group substituted with at least one fluorine atom or an alkoxy group substituted with at least one fluorine atom, and the other is a fluorine atom.

6. The production method according to any one of claims 1 to 5, wherein R³ is a fluorine atom.

7. The production method according to any one of claims 1 to 6, wherein step A is performed in the absence of a palladium catalyst or in the presence of a palladium catalyst in an amount of more than 0 mol and 0.01 mol or less, per mole of the compound represented by formula (1).

8. The production method according to any one of claims 1 to 7, wherein step A is performed in the presence of at least one solvent selected from the group consisting of ether solvents, sulfoxide solvents, nitrile solvents, and amide solvents.

9. A composition comprising
a compound represented by the following formula (3): 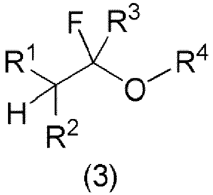 wherein
R¹ and R² each independently represent a hydrogen atom, a halogen atom, an alkyl group substituted with at least one fluorine atom, or an alkoxy group substituted with at least one fluorine atom,
R³ represents a fluorine atom, an alkyl group substituted with at least one fluorine atom, or an alkoxy group substituted with at least one fluorine atom,
R² and R³ may be bound together to form a ring, and
R⁴ represents an alkyl group optionally substituted with at least one substituent or an aryl group optionally substituted with at least one substituent; and
water.
